# EUROPEAN PATENT SPECIFICATION

(11) **EP 1 410 816 B1**
(45) Date of publication and mention of the grant of the patent: **25.02.2009**
(21) Application number: 02257162.4
(22) Date of filing: 16.10.2002
(51) Int. Cl.: A61M 5/32

(54) **Disposable syringe**
Wegwerfspritze
Seringue jetable

(43) Date of publication of application: 21.04.2004
(73) Proprietor: Shue, Ming-Jeng, Hsi Dist, Taichung City (TW); Shue, Phillip, Hsi Dist., Taichung City (TW)
(72) Inventor: Shue, Ming-Jeng, Hsi Dist, Taichung City (TW); Shue, Phillip, Hsi Dist., Taichung City (TW)
(74) Representative: Serjeants

(56) References cited:
- EP-A- 0 979 660
- EP-A- 1 216 719
- US-A1- 2002 065 489

## Description

This invention relates to a disposable syringe, more particularly to a disposable syringe which enables a needle unit to be retracted within a barrel after use for safe disposal.

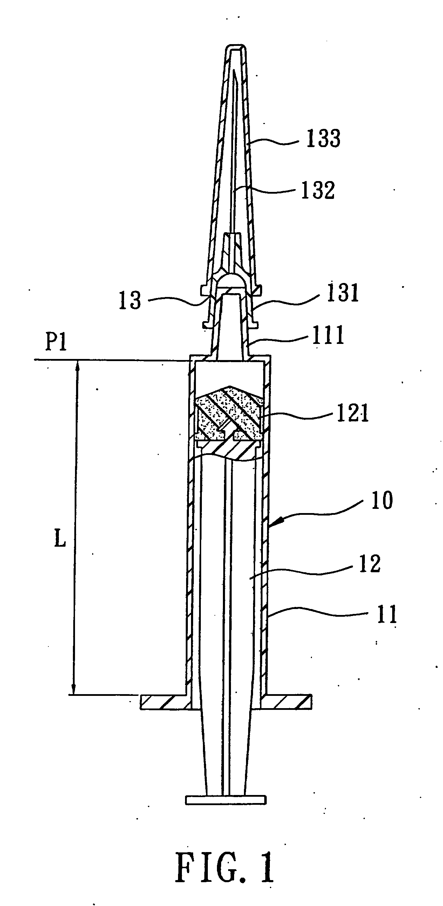

Referring to Fig. 1, a conventional disposable syringe assembly 10 is shown to include a barrel 11, a plunger 12 and a needle unit 13. The barrel 11 has an open neck end portion 111 to which a needle hub 131 of the needle unit 13 is attached, and an open rear end from which the plunger 12 is slidably inserted into the barrel 11. A forward end 121 of the plunger 12 is snugly fitted in, but is nevertheless slidable along the inner surface of the barrel 11 through a distance (L) . The needle unit 13 further has a needle 132 extending from the needle hub 131, and a cap 133 with an open end which is fitted around an outer edge of the needle hub 131, with the needle 132 enclosed within the cap 133. The cap 133 is removed from the needle hub 131 when the syringe assembly 10 is to be used, and is sleeved back after use to ensure that the needle 132 is covered. Thereafter, the needle unit 13 is detached from the barrel 11 along with the cap 133 covering the needle 132.

However, the medical or nursing personnel who has to handle the conventional disposable syringe assembly 10, is exposed to the risk of being pricked by the needle 132 when sleeving the cap 133 back on the needle hub 131 after using the disposable syringe assembly 10 since the dimension of the open end of the cap 133 is relatively small. Moreover, as forward movement of the forward end 121 of the plunger 12 terminates at the point (P1), some medicine or blood may remain within the neck end portion 111, which can cause contamination to the personnel who is unfortunately pricked by the needle 132.

A disposable syringe is disclosed in EP 0979660 A1 which is formed so that a piston with a needle support is pulled into the syringe body by pulling out the piston after a locking projection of the piston tip is inserted into and engaged with a locking hole of the needle support by pressing in the piston. A locking structure comprising interlocking protrusions for preventing the needle support from slipping off is provided between an inner peripheral face of a smaller-diameter portion of the syringe body and an outer peripheral face of the needle support. A seal structure for preventing liquid leak is formed in the locking portion of the locking structure. When a locking projection of the locking structure is inserted into and engaged with a locking hole of the locking structure, the needle support is deformed to expand the inner peripheral face of the smaller-diameter portion in the radial direction and disengage the engagement of the locking structure.

This syringe suffers from a sealing structure that is prone to leakage and an overly complicated locking structure.

Another disposable syringe is disclosed in EP 1216719 A1, the syringe having a retaining device formed on a front end of a barrel, a plurality of bosses formed on a needle hub to rotatably correspond to the retaining device, a plurality of recesses defined inside the needle hub, and a plurality of inclined teeth formed on a plunger to correspond to the recesses, such that the engagement between the retaining device and the bosses of the needle hub is able to be released by the rotational movement of the plunger together with the needle hub due to the connection of the inclined teeth and the recesses. The syringe suffers from sealing mechanism that is also liable to leakage, and a withdrawal mechanism which necessitates a rotational movement. EP 1216719 A1 also discloses in Figures 4 and 5 a conventional syringe which has a needle seat wholly contained within a narrower diameter portion of the barrel. The syringe suffers from a poor connection between seat and barrel that is liable to leak or slip as the syringe is used.

The object of the present invention is to provide a disposable syringe which provides greater safety in use and which eliminates the aforesaid drawback of the prior art.

According to this invention, the disposable syringe includes a barrel having an inner surrounding barrel wall surface which surrounds an axis and which confines a passage. The passage has lower and upper open ends disposed opposite to each other in a longitudinal direction parallel to the axis. The inner surrounding barrel wall surface includes a larger-diameter segment and a smaller-diameter segment which are disposed proximate to the lower and upper open ends, respectively, and which cooperatively form a surrounding shoulder portion therebetween.

A tubular needle seat is insertable into the passage from the lower open end toward the upper open end, and includes a lower surrounding edge portion disposed to abut against the first surrounding shoulder portion, an upper surrounding edge portion disposed opposite to the lower surrounding edge portion in the longitudinal direction, and a surrounding seat wall interposed therebetween. The surrounding seat wall has an inner tubular wall surface which surrounds the axis to confine a duct and which forms a grip segment, and an outer tubular wall surface. The outer tubular wall surface includes a lower segment proximate to the lower surrounding edge portion, and an upper segment disposed opposite to the lower segment and proximate to the upper surrounding edge portion. When the upper surrounding edge portion is forced to extend toward the upper open end after the tubular needle seat is inserted into the passage, the lower segment will be brought to a position of use, where the lower segment engages and is retained at the smaller-diameter segment by virtue of a first friction force generated therebetween while the lower surrounding edge portion abuts against the surrounding shoulder portion. In addition, when the upper surrounding edge portion is moved against the first friction force toward the lower open end passing the larger-diameter segment, the needle seat will be brought to a retracted position, where the lower segment and the lower surrounding edge portion are remote from the smaller-diameter segment and the first surrounding shoulder portion, respectively. The outer tubular wall surface is adapted to be fitted with a needle unit.

A plunger includes a stem portion which is disposed to be movable in the passage and which has inner and outer segments opposite to each other in the longitudinal direction. The outer segment extends outwardly of the lower open end of the passage. An actuated end extends from the outer segment so as to be actuated to move the stem portion along the passage. A surrounding engaging portion is retainingly sleeved on the inner segment by virtue of a second friction force, and is in sealing contact with and is slidable relative to the larger-diameter segment so as to be moved with the stem portion to the position of use. A head extends from the inner segment toward the grip segment. When the surrounding engaging portion is brought by the inner segment to engage the lower surrounding edge portion and is depressed by the lower surrounding edge portion by virtue of a third force that is generated as a consequence of continuing movement of the inner segment towards the smaller-diameter segment and that third force is greater than the second friction force, the surrounding engaging portion is retained by the lower surrounding edge portion to thereby permit the head to move towards the grip segment of the tubular needle seat and to be held by virtue of a fourth friction force that is greater than the first friction force.

Other features and advantages of the present invention will become apparent in the following detailed description of the preferred embodiments of the invention, with reference to the accompanying drawings, in which:
Fig. 1 is a longitudinal sectional view of a conventional disposable syringe assembly;
Fig. 2 is an exploded perspective view of a first preferred embodiment of a disposable syringe according to this invention for use with a needle unit to form a syringe assembly;
Fig. 3 is a perspective view of the syringe assembly according to the first preferred embodiment;
Fig. 4 is a longitudinal sectional view showing the syringe assembly during use;
Fig. 5 is a longitudinal sectional view showing the syringe assembly in a state of use;
Fig. 6 is a longitudinal sectional view showing the syringe assembly in a state in which a head of a plunger is held by a grip segment of a needle seat;
Fig. 7 is a longitudinal sectional view showing the syringe assembly in a retracted state;
Fig. 8 is a longitudinal sectional view showing a second preferred embodiment of a disposable syringe according to this invention for use with a needle unit to form a syringe assembly;
Fig. 9 is a longitudinal sectional view showing the syringe assembly according to the second preferred embodiment in a state in which a head of a plunger is held by a grip segment of a needle seat;
Fig. 10 is a longitudinal sectional view showing the syringe assembly in a retracted state;
Fig. 11 is an exploded longitudinal sectional view showing a third preferred embodiment of a disposable syringe according to this invention for use with a needle unit to form a syringe assembly;
Fig. 12 is a longitudinal sectional view showing the syringe assembly in a retracted state;
Fig. 13 is a fragmentary longitudinal sectional view showing a fourth preferred embodiment of a disposable syringe according to this invention for use with a needle unit;
Fig. 14 is a fragmentary longitudinal sectional view showing a fifth preferred embodiment of a disposable syringe according to this invention for use with a needle unit; and
Fig. 15 is a fragmentary longitudinal sectional view showing a sixth preferred embodiment of a disposable syringe according to this invention for use with a needle unit.

Before the present invention is described in greater detail, it should be noted that same reference numerals have been used to denote like elements throughout the specification.

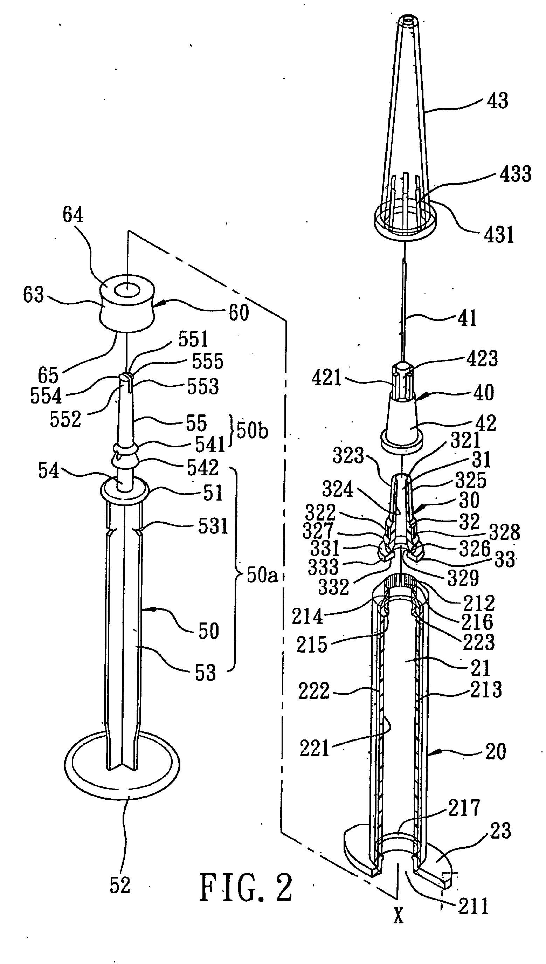

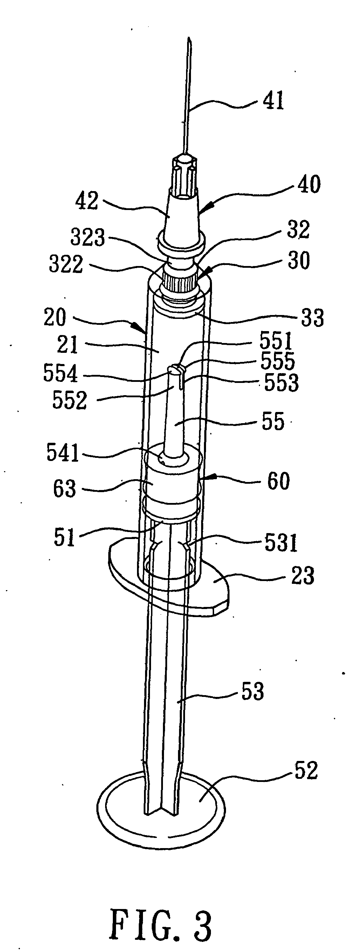

Referring to Figs. 2, 3 and 4, the first preferred embodiment of the disposable syringe according to the present invention is shown to be used with a needle unit 40 to form a syringe assembly. The needle unit 40 includes a needle hub 42 and a needle 41 which is securely affixed to the needle hub 42. The needle hub 42 has a sleeved portion 423 withapluralityof longitudinally extending frictional ribs 421 formed thereon. A tip protector 43 has an internal sleeve end 431 which is configured to be sleeved on the sleeved portion 423 for shielding the needle 41 and which has a plurality of longitudinally extending frictional ribs 433 for engaging the frictional ribs 421.

The syringe in this embodiment is shown to comprise a barrel 20, a tubular needle seat 30, and a plunger 50.

The barrel 20 has an inner surrounding barrel wall surface 221 which surrounds an axis (X) and which confines a passage 21. The passage 21 has lower and upper open ends 211,212 which are disposed opposite to each other in a longitudinal direction parallel to the axis (X). The inner surroundingbarrel wall surface 221 includes a larger-diameter segment 213 and a smaller-diameter segment 214 which are disposed proximate to the lower and upper open ends 211,212, respectively, and which cooperatively form a first surrounding shoulder portion 215 therebetween. A finger flange 23 is disposed on an outer surrounding barrel wall surface 222 of the barrel 20 at the lower open end 211. The smaller-diameter segment 214 is formed with a retaining groove 223 proximate to the first surrounding shoulder portion 215, and an axially extending friction spline portion 216 remote from the first surrounding shoulder portion 215. A protruding ring 217 is formed on and projects inwardly and radially from the larger-diameter segment 213 adjacent to the lower open end 211.

The tubular needle seat 30 is disposed to be insertable into the passage 21 from the lower open end 211 toward the upper open end 212. The needle seat 30 includes a lower surrounding edge portion 33 disposed to abut against the first surrounding shoulder portion 215 and having an outer surrounding surface 333 which abuts sealingly against the larger-diameter segment 213, an upper surrounding edge portion 321 disposed opposite to the lower surrounding edge portion 33 in the longitudinal direction, and a surrounding seat wall 32 interposed therebetween.

The seat wall 32 has an inner tubular wall surface 324 which surrounds the axis (X) to confine a duct 31. The duct 31 has a diameter that gradually decreases from the lower surrounding edge portion 33 to the upper surrounding edge portion 321. The inner tubular wall surface 324 forms a grip segment. In this embodiment, the grip segment includes a concave portion 326 which extends inwardly from the inner tubular wall surface 324 in radial directions relative to the axis (X) to form a second surrounding shoulder portion 329 that is adjacent to the lower surrounding edge portion 33.

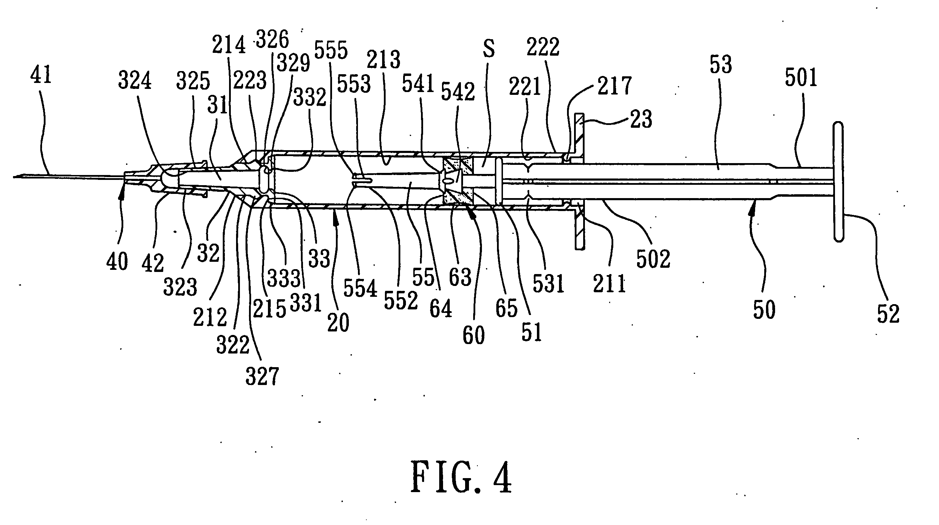

The seat wall 32 further has an outer tubular wall surface 325 which includes lower and upper segments 322,323. The lower segment 322 is proximate to the lower surrounding edge portion 33, and has a retaining protrusion 327 formed thereon. When the upper surrounding edge portion 321 is forced to extend outwardly of the upper open end 212 of the barrel 20 after the tubular needle seat 30 is inserted into the passage 21, the lower segment 322 will be brought to a position of use, as shown in Fig. 4, where the lower segment 322 engages and is retained at the smaller-diameter segment 214 by virtue of a first friction force generated as a result of retaining engagement of the retaining protrusion 327 in the retaining groove 223, while an upper edge surface 331 of the lower surrounding edge portion 33 abuts against the first surrounding shoulder portion 215 of the barrel 20. The lower segment 322 further has an axially extending friction spline portion 328 which cooperates with the friction spline portion 216 of the smaller-diameter segment 214 to form a spline member so as to prevent relative rotation between the needle seat 30 and the barrel 20. The upper segment 323 is disposed opposite to the lower segment 322 and proximate to the upper surrounding edge portion 321, and is adapted to be sleeved over by the needle hub 42 of the needle unit 40 so as to communicate the duct 31 of the needle seat 30 with the needle hub 42.

The plunger 50 includes a stem portion 50a which is movable in the passage 21 of the barrel 20. The stem portion 50a has apluralityof wingplates 53 angularlydisplaced from one another, a surrounding flange 51 disposed on upper edges of the wing plates 53, a connecting shank 54 extending from the surrounding flange 51, and a retaining protrusion 542 . The wing plates 53 are tapered downwardly. On the other hand, the stem portion 50a includes inner and outer segments 502,501 opposite to each other in the longitudinal direction. The outer segment 501 extends outwardly of the lower open end 211 of the barrel 20. An actuated end 52, which is a thumb rest, extends from the outer segment 501 so as to be actuated to move the stem portion 50a along the passage 21.

The plunger 50 further includes a head 50b which has a tapered surrounding abutting portion 541 that extends from the retaining protrusion 542 of the inner segment 502 toward the grip segment of the needle seat 30, and a spindle portion 55 which extends upwardly from the surrounding abutting portion 541 along the axis (X) and which terminates at two split halves 552,553 that are spaced apart from each other in a direction transverse to the longitudinal direction with a concave space 551 inbetween. The split halves 552,553 respectively have upper terminal anchoring edges 554,555. Preferably, the spindle portion 55 has an outer diameter which decreases from the surrounding abutting portion 541 toward the split halves 552,553. Moreover, the surrounding abutting portion 541 and the retaining protrusion 542 are configured to be substantially hollow so as to provide greater flexibility.

A deformable surrounding engaging portion 60, preferably made of an elastic material, is retainingly sleeved on the whole retaining protrusion 542 of the inner segment 502 and a portion of the surrounding abutting portion 541 to generate a second friction force. The surrounding engaging portion 60 has upper and lower end faces 64,65 opposite to each other in the longitudinal direction and proximate to and distal from the lower surrounding edge portion 33 of the needle seat 30, respectively, and an outer surrounding surface 63 disposed therebetween. The lower end face 65 is distal from the surrounding flange 51 by a space (S). The outer surrounding surface 63 sealingly contacts and is slidable relative to the larger-diameter segment 213 of the barrel 20 so as to be moved with the stem portion 50a. Preferably, the outer surrounding surface 63 is concaved toward the axis (X) to decrease contact area between the outer surrounding surface 63 and the larger-diameter segment 213 so as to facilitate sliding movement of the surrounding engaging portion 60 relative to the larger-diameter segment 213.

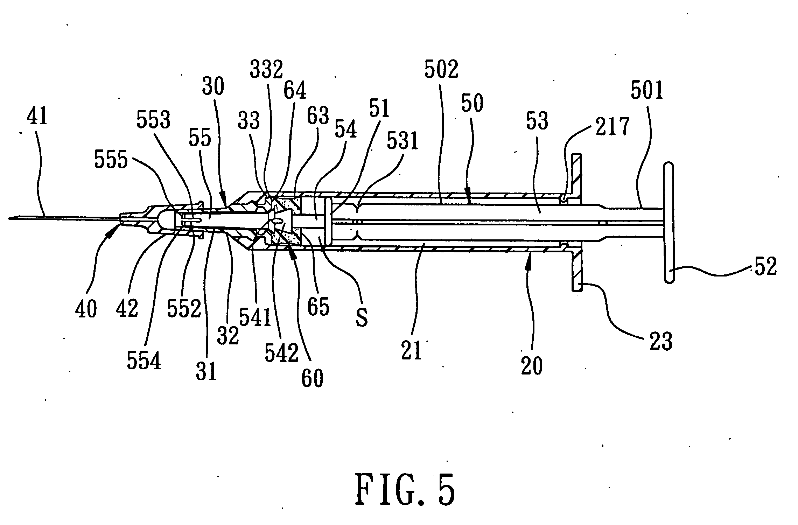

With reference to Fig. 5, in use, the plunger 50 is pressed forwardly to push the upper end face 64 of the surrounding engaging portion 60 to abut against a lower edge surface 332 of the lower surrounding edge portion 33 of the needle seat 30. Since the spindle portion 55 is configured to be tapered upwardly and to match with the inner tubular wall surface 324 of the needle seat 30, drug solution in the passage 21 can almost be completely injected via the needle unit 40.

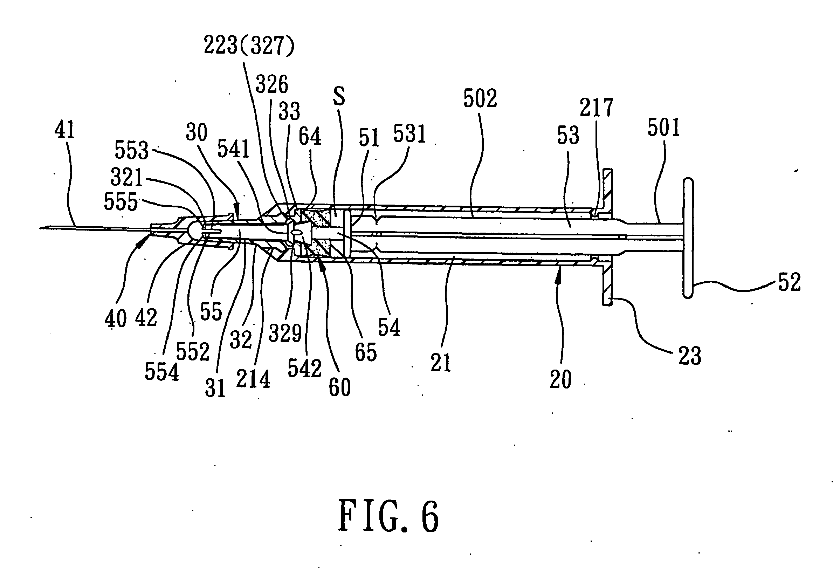

Subsequently, referring to Fig. 6, when the plunger 50 is further moved forward by a third force towards the smaller-diameter segment 214 against the second friction force between the inner segment 502 and the surrounding engaging portion 60, the surrounding engaging portion 60 is deformed by the lower surrounding edge portion 33, thereby decreasing the space (S) and permitting the surrounding abutting portion 541 of the head 50b to move in the concave portion 326 of the needle seat 30 to abut against the second surrounding shoulder portion 329.

Meanwhile, the split halves 552,553 of the spindle portion 55 are pressed by the inner tubular wall surface 324 to move towards each other against a biasing action when the split halves 552,553 are moved towards the upper surrounding edge portion 321. Once the split halves 552,553 are moved beyond the upper surrounding edge portion 321, the upper terminal anchoring edges 554,555 will engage the upper surrounding edge portion 321 to generate a friction force that combines with the friction force between the surrounding abutting portion 541 and the second surrounding shoulder portion 329 to form a fourth friction force that is greater than the first friction force between the retaining protrusion 327 and the retaining groove 223.

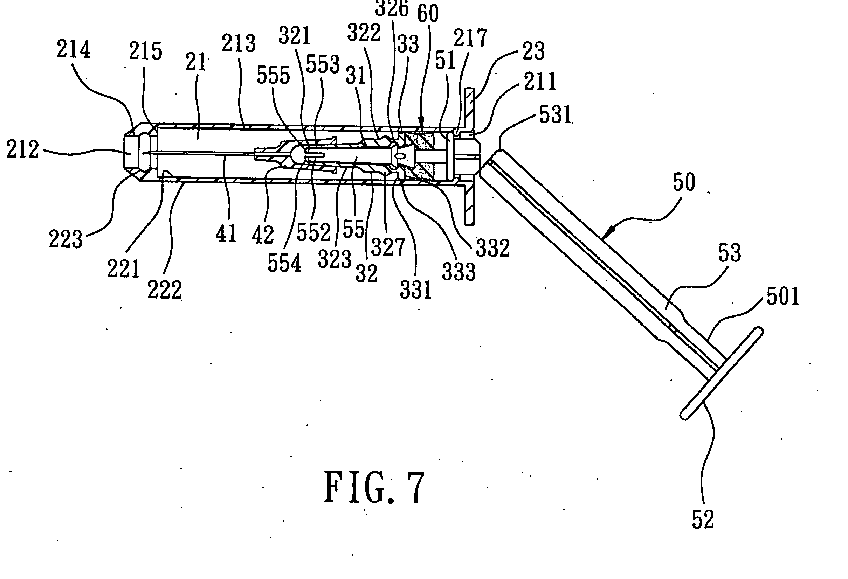

After use, referring to Fig. 7, the outer segment 501 of the plunger 50 is pulled backward to be remote from the finger flange 23 so that the needle seat 30, on which the used needle unit 40 is mounted, is retracted into the passage 21 via the upper open end 212 to bring the upper surrounding edge portion 321 to a retracted position. When the surrounding flange 51 abuts against the protruding ring 217, further movement of the plunger 50 is prevented, thereby restraining the surrounding engaging portion 60 from being moved out of the lower open end 211. In this state, the used needle unit 40 can be enclosed in the passage 21 of the barrel 20 for disposal.

Consequently, chances that the user may be accidentally pricked or pierced by the needle 41 are slim. The safety in use and disposal is thus enhanced.

Furthermore, the wing plates 53 at the inner segment 502 have a weakening area 531 disposed proximate to the surrounding flange 51. As such, when the plunger 50 is retracted to push the surrounding flange 51 to abut against the protruding ring 217, the weakening area 531 extends outwardly of the lower open end 211 so as to ease breaking of the stem portion 50a off the plunger 50 at the weakening area 531 for convenient disposal.

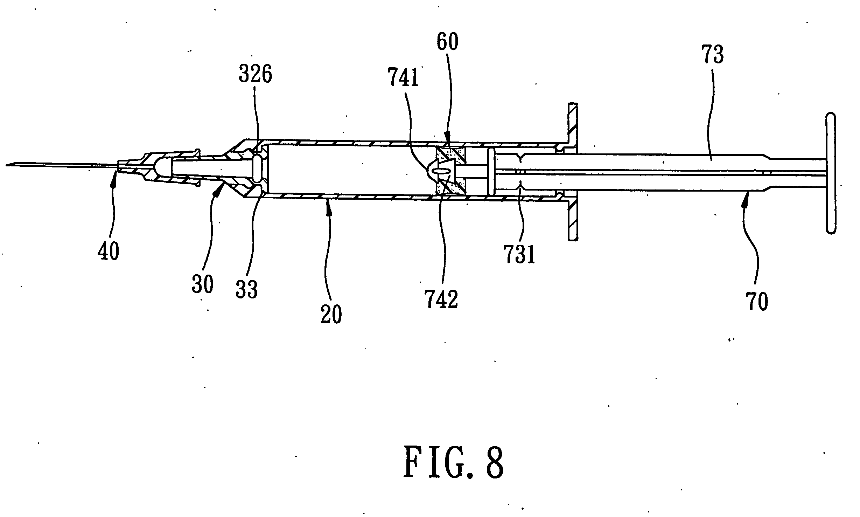

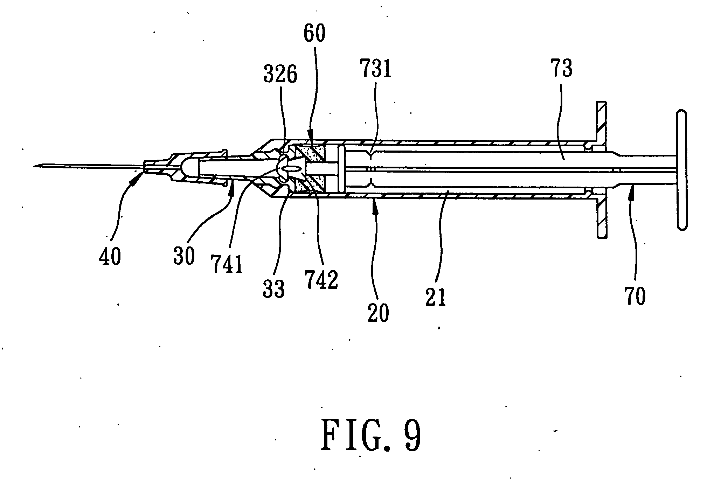

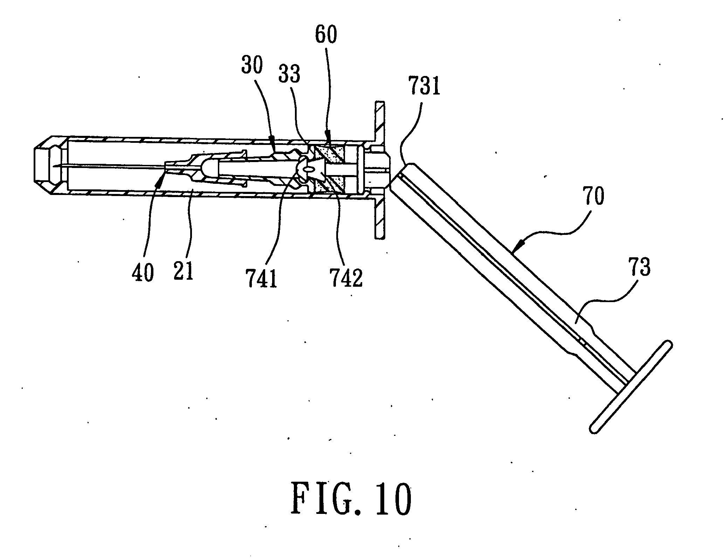

Referring to Fig. 8, the second preferred embodiment of the disposable syringe according to this invention is shown to be similar to the aforesaid embodiment in construction. The differences therebetween reside in that the spindle portion in the aforesaid embodiment is dispensed with. An elastic surrounding engaging portion 60 is retainingly sleeved on the whole retaining protrusion 742 and a portion of the surrounding abutting portion 741. As such, when the surrounding engaging portion 60 is brought by the stem portion 73 of the plunger 70 upwardly to engage and to be depressed by the lower surrounding edge portion 33 of the needle seat 30, the surrounding engaging portion 60 is retained by the lower surrounding edge portion 33, thereby permitting the surrounding abutting portion 741 to be retained in the concave portion 326, as shown in Fig. 9. Hence, the needle seat 30 and the needle unit 40 can be retracted into the passage 21 of the barrel 20 after an injection procedure to permit breaking of the stem portion 73 of the plunger 70 at a weakening area 731 for safe disposal, as shown in Fig. 10.

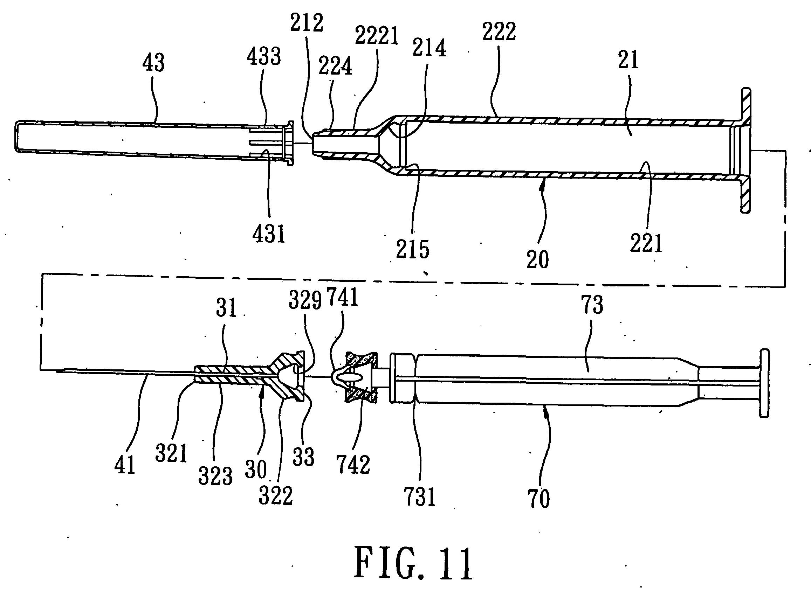

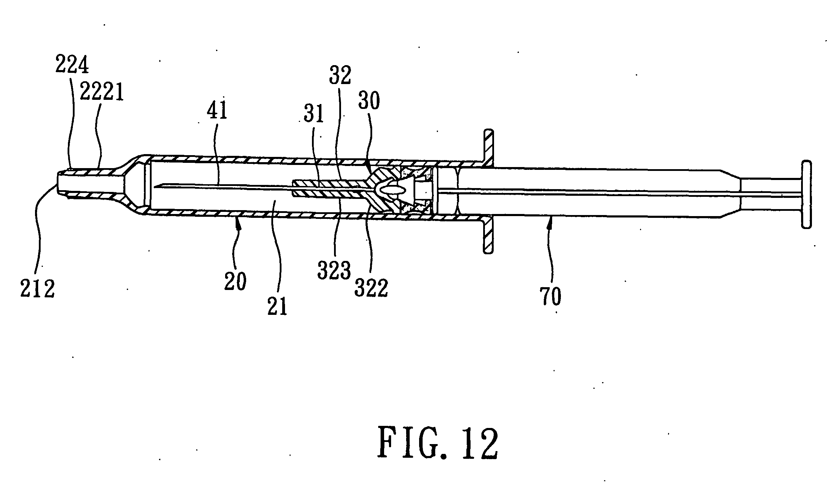

Referring to Figs. 11 and 12, the third preferred embodiment of the disposable syringe according to this invention is shown to be similar to the aforesaid embodiments in construction. The differences therebetween reside in that a tubular needle seat 30 has a duct 31 with a relatively small diameter at the upper segment 323 so as to engage fittingly the needle 41. Thus, the needle hub 42 can be eliminated.

Moreover, a barrel 20 has an outer surrounding wall surface 222 with a sleeved portion 2221 which is disposed proximate to the upper open end 212 to replace the sleeved portion 423 shown in Fig. 2, and which has a plurality of friction ribs 224 formed thereon. Thus, a tip protector 43, which has a plurality of frictional ribs 433 formed on an internal sleeve end 431 thereof, can be sleeved fittingly on the sleeved portion 2221 for shielding the needle 41.

In the syringe assembly of this embodiment, the number of components is decreased. Similarly, the needle seat 30 and the needle 41 can be retracted into the passage 21 of the barrel 20 after an injection procedure.

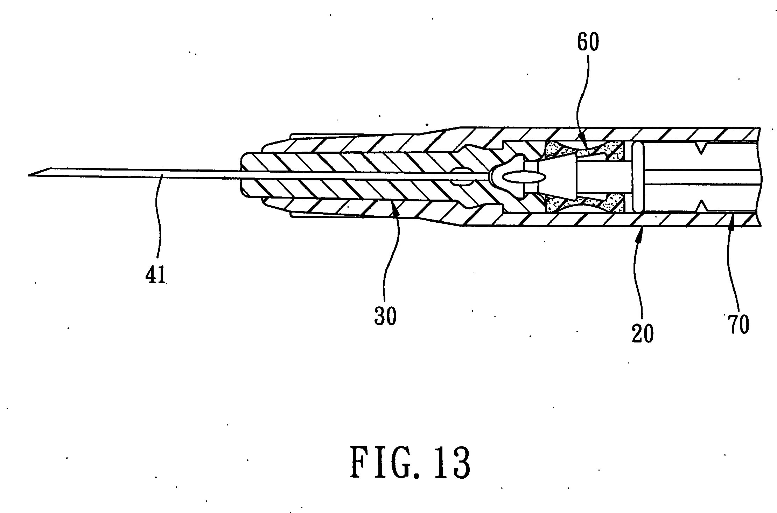

Referring to Fig. 13, the fourth preferred embodiment of the disposable syringe with a similar construction of the aforesaid embodiments, is used for an extremely small injection volume, such as 1 ml. Thus, the dimensions of the barrel 20, the needle seat 30, the needle 41, the plunger 70, and the deformable surrounding engaging portion 60 are smaller than those of the aforesaid embodiments.

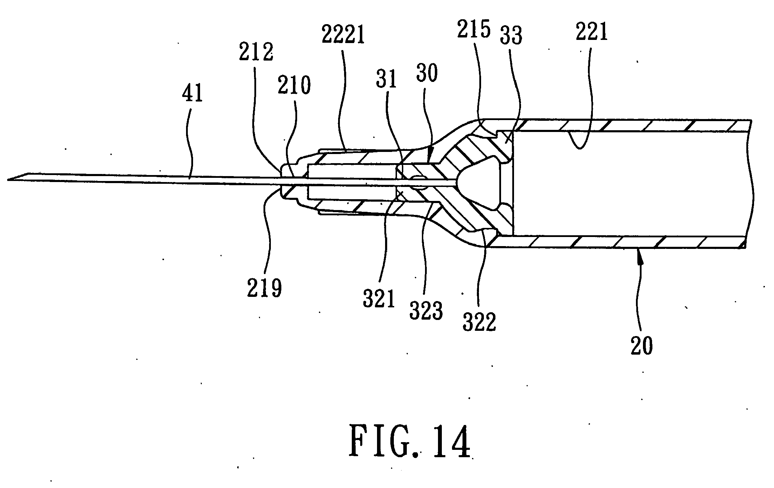

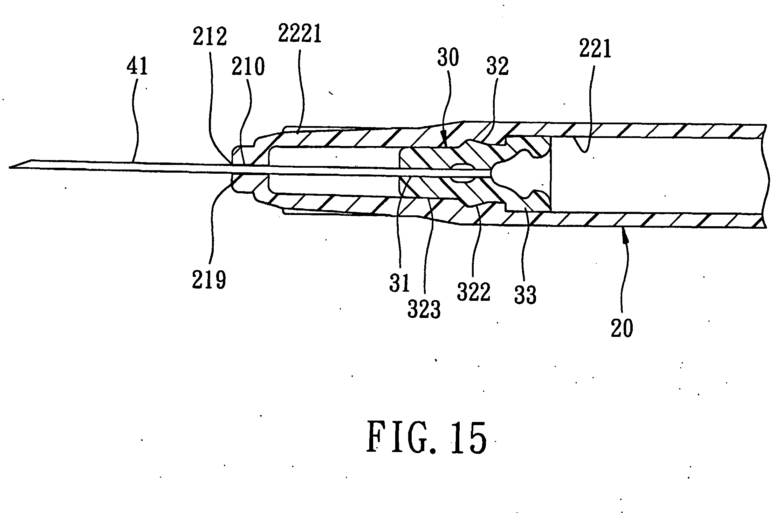

Referring to Figs. 14 and 15, the fifth and sixth preferred embodiments of the disposable syringe according to this invention are shown to be similar to the third and fourth embodiments in construction, respectively. The differences therebetween reside in that the upper open end 212 has a surrounding edge wall 219 which extends from the smaller-diameter segment 214 of the inner surrounding barrel wall surface 221 of the barrel 20 toward the axis (X) and which confines a through hole 210 that surrounds the axis (X). As such, the needle 41 can pass through the through hole 210 and outwardly of the upper open end 212. The sixth preferred embodiment shown in Fig. 15 is similar to the fifth preferred embodiment in construction, but is used for an extremely small injection volume, such as 1 ml.

## Claims

1. A disposable syringe adapted to be used with a needle unit (40), comprising:
a barrel (20) having an inner surrounding barrel wall surface (221) which surrounds an axis (X) and which confines a passage (21), said passage (21) having lower and upper open ends (211,212) which are disposed opposite to each other in a longitudinal direction parallel to the axis, and
a plunger (50,70) including
a stem portion (50a,73) disposed to be movable in said
passage (21) of said barrel (20), and having inner and outer segments (502,501) opposite to each other in the longitudinal direction, said outer segment (501) extending outwardly of said lower open end (211), and
an actuated end (52) extending from said outer segment (501) so as to be actuated to move said stemportion (50a, 73) along said passage (21), whereby,
said inner surrounding barrel wall surface (221) of said barrel (20) includes a larger-diameter segment (213) and a smaller-diameter segment (214) which are disposed proximate to said lower and upper open ends (211, 212), respectively, and which cooperatively form a first surrounding shoulder portion (215) therebetween;
said disposable syringe further comprising:
a tubular needle seat (30) disposed to be insertable into said passage (21) from said lower open end (211) toward said upper open end (212), and adapted to be fitted with the needle unit (40), said needle seat (30) including
a lower surrounding edge portion (33) which is disposed to abut against said first surrounding shoulder portion (215),
an upper surrounding edge portion (321) disposed opposite to said lower surrounding edge portion (33) in the longitudinal direction, and
a surrounding seat wall (32) interposed said lower and upper surrounding edge portions (33,321), and having an inner tubular wall surface (324) which surrounds the axis (X) to confine a duct (31) and which has a grip segment, and an outer tubular wall surface (325) which includes a lower segment (322) proximate to said lower surrounding edge portion (33), and configured such that, when said upper surrounding edge portion (321) is forced to extend toward said upper open end (212) after said tubular needle seat (30) is inserted into said passage (21), said lower segment (322) will be brought to a position of use, where said lower segment (322) engages and is retained at said smaller-diameter segment (214) by virtue of a first friction force generated therebetween while said lower surrounding edge portion (33) abuts against said first surrounding shoulder portion (215), and such that, when said upper surrounding edge portion (321) is moved against the first friction force toward said lower open end (211) passing said larger-diameter segment (213), said needle seat (30) will be brought to a retracted position, where said lower segment (322) and said lower surrounding edge portion (33) are remote from said smaller-diameter segment (214) and said first surrounding shoulder portion (215), respectively, and
an upper segment (323) disposed opposite to said lower segment (322) and proximate to said upper surrounding edge portion (321);
said plunger (50,70) further including
a surrounding engaging portion (60) which is disposed to retainingly sleeve on said inner segment (502) by virtue of a second friction force, and which is in sealing contact with and which is slidable relative to said larger-diameter segment (213) so as to be moved with said stem portion (50a,73) to the position of use, and
a head (50b) disposed to extend from said inner segment (502) toward said grip segment of said inner tubular wall surface (324), and configured such that, when said surrounding engaging portion (60) is brought by said inner segment (502) to engage said lower surrounding edge portion (33) and is depressed by said lower surrounding edge portion (33) by virtue of a third force generated as a consequence of continuing movement of said inner segment (502) towards said smaller-diameter segment (214), the third force being greater than the second friction force, said surrounding engaging portion (60) is retained by said lower surrounding edge portion (33) to thereby permit said head (50b) to move towards said grip segment and to be held by virtue of a fourth friction force that is greater than the first friction force.

2. The disposable syringe of Claim 1, further **characterized in that** one of said lower segment (322) and said smaller-diameter segment (214) is formed with a retaining groove (223), and the other one of said lower segment (322) and said smaller-diameter segment (214) is formed with a retaining protrusion (327) which engages retainingly said retaining groove (223) when said lower segment (322) is brought to the position of use so as to generate the first friction force.

3. The disposable syringe of Claim 1, further **characterized in that** said grip segment has a concave portion (326) which extends inwardly from said inner tubular wall surface (324) in radial directions relative to the axis to form a second surrounding shoulder portion (329) that is adjacent to said lower surrounding edge portion (33), said head (50b) having a surrounding abutting portion (541, 741) which is configured so as to be insertable into said concave portion (326) and to abut against said second surrounding shoulder portion (329), thereby generating the fourth friction force to hold said needle seat (30) at the retracted position.

4. The disposable syringe of Claim 3, further **characterized in that** said head (50b) has a spindle portion (55) which extends from said surrounding abutting portion (541) along the axis (X) and which terminates at two split halves (552,553) that are spaced apart from each other in a direction transverse to the longitudinal direction, said split halves (552,553) respectively having two upper terminal anchoring edges (554,555) which are remote from said surrounding abutting portion (541) and which are configured such that said split halves (552,553) are pressed by said inner tubular wall surface (324) to move towards each other against a biasing action when said split halves (552,553) are moved towards said upper surrounding edge portion (321), and such that said upper terminal anchoring edges (554,555) engage said upper surrounding edge portion (321) once said split halves (552,553) are moved beyond said upper surrounding edge portion (321) .

5. The disposable syringe of Claim 4, further **characterized in that** said inner tubular wall surface (324) has a diameter which gradually decreases from said lower surrounding edge portion (33) to said upper surrounding edge portion (321), said spindle portion (55) having an outer diameter which gradually decreases from said surrounding abutting portion (541) to said split halves (552,553).

6. The disposable syringe of Claim 3, further **characterized in that** said surrounding engaging portion (60) is made of a deformable material, and has upper and lower end faces (64,65) opposite to each other in the longitudinal direction and proximate to and distal from said lower surrounding edge portion (33), respectively, and an outer surrounding surface (63) disposed between said upper and lower end faces (64, 65) and concaved toward the axis (X).

7. The disposable syringe of Claim 1, further **characterized in that** said lower surrounding edge portion (33) has an upper edge surface (331) which abuts against said first surrounding shoulder portion (215) when said lower segment (322) is at the position of use, and a lower edge surface (332) for abutment and depression by said surrounding engaging portion (60).

8. The disposable syringe of Claim 1, further **characterized by** a spline member (216,328) disposed between said smaller-diameter segment (214) and said lower segment (322) to prevent relative rotation therebetween.

9. The disposable syringe of Claim 1, further **characterized by** a protruding ring (217) which is disposed on and which projects inwardly and radially from said larger-diameter segment (213) adjacent to said lower open end (211), and a surrounding flange (51) which is disposed to surround said inner segment (502) adjacent to said head (50b) and which is engageable with said protruding ring (217).

10. The disposable syringe of Claim 9, further **characterized in that** said inner segment (502) has a weakening area (531,731) which is disposed proximate to said surrounding flange (51) such that when said surrounding flange (51) is brought to engage said protruding ring (217), said weakening area (531,731) extends outwardly of said lower open end (211) so as to ease breaking of said stem portion (50a,73) off said plunger (50,70) at said weakening area (531,731).

11. The disposable syringe of Claim 3, further **characterized in that** said surrounding abutting portion (541,741) is configured to be substantially hollow for greater flexibility.

12. The disposable syringe of Claim 1, further **characterized in that** said barrel (20) has an outer surrounding wall surface (222) disposed opposite to said inner surrounding barrel wall surface (221) in radial directions and having a sleeved portion (2221) which is disposed proximate to said upper open end (212), said disposable syringe further comprising a tip protector (43) which has an internal sleeve end (433) configured to be sleeved on said sleeved portion (2221) so as to shield a needle (41) of the needle unit (40) .

13. The disposable syringe of Claim 12, further **characterized in that** each of said sleeved portion (2221) and said internal sleeve end (433) has a plurality of friction ribs (224,433) formed thereon.

14. The disposable syringe of Claim 13, further **characterized in that** said upper open end (212) has a surrounding edge wall (219) which extends from said smaller-diameter segment (214) of said inner surrounding barrel wall surface (221) toward the axis (X) and which confines a through hole (210) that surrounds the axis (X) and that is adapted for passage of the needle (41) of the needle unit (40) .

15. The disposable syringe of Claim 1, further **characterized in that** said upper segment (323) and said upper surrounding edge portion (321) extend outwardly of said upper open end (212) when said lower segment (322) is in the position of use, said disposable syringe further comprising a needle hub (42) which is adapted to secure a needle (41) of the needle unit (40), which is disposed to sleeve over said upper segment (323), and which has a sleeved portion (423), and a tip protector (43) which has an internal sleeve end (433) configured to be sleeved on said sleeved portion (423) so as to shield the needle (41).

## Patentansprüche

1. Wegwerfspritze, die zum Verwenden mit einer Nadeleinheit (40) geeignet ist, umfassend:
einen Zylinder (20) mit einer eine Achse (X) umgebenden inneren umlaufenden Zylinderwandfläche (221), die einen Durchgang begrenzt (21), wobei der Durchgang (21) in einer Längsrichtung parallel zu der Achse einander entgegengesetzt angeordnete untere und obere offene Enden (211, 212) aufweist, und
einen Kolben (50, 70) mit
einem Schaftabschnitt (50a, 73), der dazu angeordnet ist, in dem Durchgang (21) des Zylinders (20) bewegbar zu sein, mit einander in der Längsrichtung entgegengesetzten inneren und äußeren Segmenten (502, 501), wobei sich das äußere Segment (501) von dem unteren offenen Ende (211) nach außen erstreckt, und
einem Betätigungsende (52), das sich von dem äußeren Segment (501) derart erstreckt, dass es betätigt wird, um den Schaftabschnitt (50a, 73) entlang des Durchgangs (21) zu bewegen, wobei
die innere umlaufende Zylinderwandfläche (221) des Zylinders (20) ein Segment mit größerem Durchmesser (213) und ein Segment mit kleinerem Durchmesser (214) umfasst, die nahe des unteren beziehungsweise des oberen offenen Endes (211, 212) angeordnet sind, und die zusammenwirkend dazwischen einen ersten Schulterbereich (215) bilden;
wobei die Wegwerfspritze ferner umfasst:
einen rohrförmigen Nadelsitz (30), der dazu angeordnet ist, von dem unteren offenen Ende (211) in Richtung zu dem oberen offenen Ende (212) hin in den Durchgang (21) einführbar zu sein, und der dazu geeignet ist, mit der Nadeleinheit (40) eingepasst zu werden, wobei der Nadelsitz (30) umfasst:
einen unteren umlaufenden Randbereich (33), der dazu angeordnet ist, an den ersten umlaufenden Schulterbereich (215) anzugrenzen,
einen oberen umlaufenden Randbereich (321), der dem unteren umlaufenden Randbereich (33) in der Längsrichtung gegenüber angeordnet ist, und
eine zwischen dem unteren und oberen umlaufenden Randbereich (33, 321) angeordnete umlaufende Sitzwand (32), die eine innere rohrförmige Wandfläche (324) aufweist, welche die Achse (X) umgibt, um eine Führung (31) zu begrenzen, und ein Griffsegment aufweist, und
eine äußere rohrförmige Wandfläche (325) mit
einem unteren Segment (322) nahe dem unteren umlaufenden Randbereich (33), das dazu ausgelegt ist, dass dann, wenn der obere umlaufende Randbereich (321) dazu gebracht wird, sich in Richtung auf das obere offene Ende (212) hin zu erstrecken, nachdem der rohrförmige Nadelsitz (30) in den Durchgang (21) eingefügt ist, das untere Segment (322) in eine Benutzungsstellung gebracht wird, in der das untere Segment (322) bei dem Segment mit kleinerem Durchmesser (214) in Eingriff gerät und durch eine dazwischen erzeugte erste Reibungskraft zurückgehalten wird, während der untere umlaufende Randbereich (33) an den ersten umlaufenden Schulterbereich (215) angrenzt, und dass dann, wenn der obere umlaufende Randbereich (321) gegen die erste Reibungskraft in Richtung auf das untere offene Ende (211) hin bewegt wird, der Nadelsitz (30), das Segment mit größerem Durchmesser (213) passierend, zu einer zurückgezogenen Stellung gebracht wird, in der das untere Segment (322) und der untere umlaufende Randbereich (33) von dem Segment mit kleinerem Durchmesser (214) beziehungsweise dem ersten umlaufenden Schulterbereich (215) entfernt sind, und
ein entgegengesetzt zu dem unteren Segment (322) und nahe dem oberen umlaufenden Randbereich (321) angeordnetes oberes Segment (323);
wobei der Kolben (50, 70) ferner umfasst:
einen umlaufenden Eingriffsbereich (60), der dazu angeordnet ist, durch eine zweite Reibungskraft das innere Segment (502) zurückhaltend zu umfassen, und der in abdichtenden Kontakt mit dem Segment mit größerem Durchmesser (213) steht und relativ zu diesem verschiebbar ist, um mit dem Schaftabschnitt (50a, 73) in die Benutzungsstellung bewegt zu werden, und
ein Kopfelement (50b), das dazu angeordnet ist, sich von dem inneren Segment (502) in Richtung zu dem Griffsegment der inneren rohrförmigen Wandfläche (324) hin zu erstrecken, und derart ausgelegt ist, dass dann, wenn der umlaufende Eingriffsbereich (60) durch das innere Segment (502) dazu gebracht wird, mit dem unteren umlaufenden Randbereich (33) in Eingriff zu geraten und durch den unteren umlaufenden Randbereich (33) durch eine als Folge fortgesetzter Bewegung des inneren Segments (502) in Richtung zu dem Segment mit kleinerem Durchmesser (214) hin erzeugten dritten Kraft heruntergedrückt wird, wobei die dritte Kraft größer ist als die zweite Reibungskraft, der umlaufende Eingriffsbereich (60) durch den unteren umlaufenden Randbereich (33) zurückgehalten wird, um dadurch zu erlauben, dass sich das Kopfelement (50b) in Richtung auf das Griffsegment hin bewegt und durch eine vierte Reibungskraft gehalten wird, die größer ist als die erste Reibungskraft.

2. Wegwerfspritze nach Anspruch 1, ferner **gekennzeichnet dadurch, dass** das untere Segment (322) oder das Segment mit kleinerem Durchmesser (214) mit einer Rückhaltevertiefung (223) ausgebildet ist, und das andere von dem unteren Segment (322) und dem Segment mit kleinerem Durchmesser (214) mit einem Rückhaltevorsprung (327) ausgebildet ist, der mit der Rückhaltevertiefung (223) zurückhaltend in Eingriff gerät, wenn das untere Segment (322) in die Benutzungsstellung gebracht wird, um die erste Reibungskraft zu erzeugen.

3. Wegwerfspritze nach Anspruch 1, ferner **gekennzeichnet dadurch, dass** das Griffsegment einen konkaven Bereich (326) aufweist, der sich von der inneren rohrförmigen Wandfläche (324) in radialen Richtungen relativ zu der Achse nach innen erstreckt, um einen dem unteren umlaufenden Randbereich (33) naheliegenden zweiten umlaufenden Schulterbereich (329) auszubilden, wobei das Kopfelement (50b) einen umlaufenden Anstoßbereich (541, 741) aufweist, der dazu ausgelegt ist, in den konkaven Bereich (326) einführbar zu sein und gegen den zweiten umlaufenden Schulterbereich (329) anzustoßen, um dadurch die vierte Reibungskraft zu erzeugen, um den Nadelsitz (30) bei der zurückgezogenen Stellung zu halten.

4. Wegwerfspritze nach Anspruch 3, ferner **dadurch gekennzeichnet, dass** das Kopfelement (50b) einen Spindelabschnitt (55) aufweist, der sich von dem umlaufenden Anstoßbereich (541) entlang der Achse (X) erstreckt und bei zwei geteilten Hälften (552, 553) endet, die in einer Richtung quer zu der Längsrichtung voneinander beabstandet sind, wobei die geteilten Hälften (552, 553) jeweils zwei obere Endverankerungsränder (554, 555) aufweisen, die von dem umlaufenden Anstoßbereich (541) entfernt sind und die derart ausgelegt sind, dass die geteilten Hälften (552, 553) durch die innere rohrförmige Wandfläche (324) dazu gedrängt werden, sich gegen eine Vorspannwirkung aufeinander zu zu bewegen, wenn die geteilten Hälften (552, 553) in Richtung auf den oberen umlaufenden Randbereich (321) hin bewegt werden, und dass die oberen Endverankerungsränder (554, 555) mit dem oberen umlaufenden Randbereich (321) in Eingriff stehen, sobald die geteilten Hälften (552, 553) über den oberen umlaufenden Randbereich (321) hinaus bewegt werden.

5. Wegwerfspritze nach Anspruch 4, ferner **dadurch gekennzeichnet, dass** die innere rohrförmige Wandfläche (324) einen Durchmesser aufweist, der sich von dem unteren umlaufenden Randbereich (33) zu dem oberen umlaufenden Randbereich (321) graduell verringert, wobei der Spindelabschnitt (55) einen äußeren Durchmesser aufweist, der sich von dem umlaufenden Anstoßbereich (541) zu den geteilten Hälften (552, 553) graduell verringert.

6. Wegwerfspritze nach Anspruch 3, ferner **dadurch gekennzeichnet, dass** der umlaufende Eingriffsbereich (60) aus einem deformierbaren Material hergestellt ist, und sich in der Längsrichtung entgegengesetzte obere und untere Endflächen (64, 65) aufweist, die dem unteren umlaufenden Randbereich (33) nahe beziehungsweise entfernt davon angeordnet sind, sowie eine zwischen der oberen und unteren Endfläche (64, 65) angeordnete äußere umlaufende Fläche (63) aufweist, die konkav zu der Achse (X) hin ausgebildet ist.

7. Wegwerfspritze nach Anspruch 1, ferner **dadurch gekennzeichnet, dass** der untere umlaufende Randbereich (33) eine obere Randfläche (331) aufweist, die an den ersten umlaufenden Schulterbereich (215) grenzt, wenn das untere Segment (322) sich in der Benutzungsstellung befindet, sowie eine untere Randfläche (332) zum Anstoßen und Niederdrücken durch den umlaufenden Eingriffsbereich (60).

8. Wegwerfspritze nach Anspruch 1, ferner **gekennzeichnet durch** ein Keilelement (216, 328), das zwischen dem Segment mit kleinerem Durchmesser (214) und dem unteren Segment (322) angeordnet ist, um eine relative Drehung zwischen diesen zu verhindern.

9. Wegwerfspritze nach Anspruch 1, ferner **gekennzeichnet durch** einen vorstehenden Ring (217), der nahe dem unteren offenen Ende (211) an dem Segment mit größerem Durchmesser (213) angeordnet ist und sich davon nach innen und radial erstreckt, und einen umlaufenden Flansch (51), der dazu angeordnet ist, das innere Segment (502) nahe dem Kopfelement (50b) zu umfassen, und der in Eingriff mit dem vorstehenden Ring (217) gebracht werden kann.

10. Wegwerfspritze nach Anspruch 9, ferner **dadurch gekennzeichnet, dass** das innere Segment (502) einen Schwächungsbereich (531, 731) aufweist, der nahe dem umlaufenden Flansch (51) derart angeordnet ist, dass dann, wenn der umlaufende Flansch (51) dazu gebracht wird, mit dem vorstehenden Ring (217) in Eingriff zu gelangen, sich der Schwächungsbereich (531, 731) von dem unteren offenen Ende (211) derart nach außen erstreckt, dass bei dem Schwächungsbereich (531, 731) das Abbrechen des Schaftabschnitts (50a, 73) von dem Kolben (50, 70) erleichtert ist.

11. Wegwerfspritze nach Anspruch 3, ferner **dadurch gekennzeichnet, dass** der umlaufende Anstoßbereich (541, 741) dazu ausgelegt ist, für eine größere Flexibilität im Wesentlichen hohl zu sein.

12. Wegwerfspritze nach Anspruch 1, ferner **dadurch gekennzeichnet, dass** der Zylinder (20) eine äußere umlaufende Wandfläche (222) aufweist, die in radialen Richtungen gegenüber der inneren umlaufenden Zylinderwandfläche (221) angeordnet ist und einen nahe des oberen offenen Endes (212) angeordneten Umhüllungsbereich (2221) aufweist, wobei die Wegwerfspritze ferner eine Spitzenschutzeinrichtung (43) aufweist, die ein inneres Hüllenende (433) umfasst, das dazu ausgelegt ist, auf dem Umhüllungsbereich (2221) angeordnet zu werden, um eine Nadel (41) der Nadeleinheit (40) zu schützen.

13. Wegwerfspritze nach Anspruch 12, ferner **dadurch gekennzeichnet, dass** sowohl der Umhüllungsbereich (2221) als auch das innere Hüllenende (433) mehrere darauf ausgebildete Reibungsrippen (224, 433) aufweisen.

14. Wegwerfspritze nach Anspruch 13, ferner **dadurch gekennzeichnet, dass** das obere offene Ende (212) eine umlaufende Randwand (219) aufweist, die sich von dem Segment mit kleinerem Durchmesser (214) der inneren umlaufenden Zylinderwandfläche (221) in Richtung zu der Achse (X) hin erstreckt und ein Durchgangsloch (210) begrenzt, welches die Achse (X) umfasst und für einen Durchgang der Nadel (41) der Nadeleinheit (40) geeignet ist.

15. Wegwerfspritze nach Anspruch 1, ferner **dadurch gekennzeichnet, dass** das obere Segment (323) und der obere umlaufende Randbereich (321) sich von dem oberen offenen Ende (212) nach außen erstrecken, wenn das untere Segment (322) sich in der Benutzungsstellung befindet, wobei die Wegwerfspritze ferner eine Nadelsitzvorrichtung (42) umfasst, die dazu geeignet ist, eine Nadel (41) der Nadeleinheit (40) zu sichern, die dazu angeordnet ist, das obere Segment (323) zu umhüllen, und einen Umhüllungsbereich (423) und eine Spitzenschutzeinrichtung (43) mit einem inneren Hüllenende (433) aufweist, das dazu ausgelegt ist, auf dem Umhüllungsbereich (423) angeordnet zu sein, um die Nadel (41) zu schützen.

## Revendications

1. Seringue jetable adaptée pour être utilisée avec une unité d'aiguille (40), comprenant :
un tube (20) ayant une surface de paroi de tube environnante interne (221) qui entoure un axe (X) et qui confine un passage (21), ledit passage (21) ayant des extrémités ouvertes inférieure et supérieure (211, 212) qui sont disposées à l'opposé l'une de l'autre dans une direction longitudinale parallèle à l'axe, et
un plongeur (50, 70) comprenant
une portion de tige (50a, 73) disposée pour être mobile dans ledit passage (21) dudit tube (20), et ayant des segments interne et externe (502, 501) opposés l'un à l'autre dans la direction longitudinale, ledit segment externe (501) s'étendant vers l'extérieur de ladite extrémité ouverte inférieure (211), et
une extrémité actionnée (52) s'étendant depuis ledit segment externe (501) de façon à être actionnée pour déplacer ladite portion de tige (50a, 73) le long dudit passage (21),
ladite surface.de paroi de tube environnante interne (221) dudit tube (20) comprenant un segment de plus grand diamètre (213) et un segment de plus petit diamètre (214) qui sont disposés à proximité desdites extrémités ouvertes inférieure et supérieure (211, 212), respectivement, et qui forment de manière coopérante une première portion d'épaulement environnante (215) entre elles ;
ladite seringue jetable comprenant en outre :
un siège d'aiguille tubulaire (30) disposé pour être insérable dans ledit passage (21) à partir de ladite extrémité ouverte inférieure (211) vers ladite extrémité ouverte supérieure (212) et adapté pour être ajusté avec l'unité d'aiguille (40), ledit siège d'aiguille (30) comprenant
une portion de bord environnante inférieure (33) qui est disposée pour venir en butée contre ladite première portion d'épaulement environnante (215),
une portion de bord environnante supérieure (321) disposée à l'opposé de ladite portion de bord environnante inférieure (33) dans la direction longitudinale, et
une paroi de siège environnante (32) interposée entre lesdites portions de bord environnante inférieure et supérieure (33, 321), et ayant une surface de paroi tubulaire interne (324) qui entoure ledit axe (X) pour confiner un conduit (31) et qui a un segment de saisie, et une surface de paroi tubulaire externe (325) qui comprend
un segment inférieur (322) à proximité de ladite portion de bord environnante inférieure (33), et configuré de telle sorte que, lorsque ladite portion de bord environnante supérieure (321) est forcée pour s'étendre vers ladite extrémité ouverte supérieure (212) après insertion dudit siège d'aiguille tubulaire (30) dans ledit passage (21), ledit segment inférieur (322) sera amené vers une position d'utilisation, où ledit segment inférieur (322) s'engage et est retenu au niveau dudit segment de plus petit diamètre (214) en vertu d'une première force de frottement générée entre eux alors que ladite portion de bord environnante inférieure (33) vient en butée contre ladite première portion d'épaulement environnante (215), et de telle sorte que, lorsque ladite portion de bord environnante supérieure (321) est déplacée contre la première force de frottement vers ladite extrémité ouverte inférieure (211) passant par ledit segment de plus grand diamètre (213), ledit siège d'aiguille (30) sera amené vers une position rétractée, où ledit segment inférieur (322) et ladite portion de bord environnante inférieure (33) sont éloignés dudit segment de plus petit diamètre (214) et de ladite première portion d'épaulement environnante (215), respectivement, et
un segment supérieur (323) disposé à l'opposé dudit segment inférieur (322) et à proximité de ladite portion de bord environnante supérieure (321) ;
ledit plongeur (50, 70) comprenant en outre
une portion d'engagement environnante (60) qui est disposée pour se manchonner avec faculté de retenue sur ledit segment interne (502) en vertu d'une deuxième force de frottement, et qui est en contact d'étanchéité avec et peut glisser par rapport audit segment de plus grand diamètre (213) de façon à être déplacée avec ladite portion de tige (50a, 73) vers la position d'utilisation, et
une tête (50b) disposée pour s'étendre depuis ledit segment interne (502) vers ledit segment de saisie de ladite surface de paroi tubulaire interne (324), et configurée de telle sorte que, lorsque ladite portion d'engagement environnante (60) est amenée par ledit segment interne (502) à s'engager avec ladite portion de bord environnante inférieure (33) et est enfoncée par ladite portion de bord environnante inférieure (33) en vertu d'une troisième force générée par suite d'un mouvement continu dudit segment interne (502) vers ledit segment de plus petit diamètre (214), la première force étant plus grande que la deuxième force de frottement, ladite portion d'engagement environnante (60) est retenue par ladite portion de bord environnante inférieure (33) pour permettre ainsi à ladite tête (50b) de se déplacer vers ledit segment de saisie et d'être maintenue en vertu d'une quatrième force de frottement plus grande que la première force de frottement.

2. Seringue jetable selon la revendication 1, **caractérisée en outre en ce que** l'un dudit segment inférieur (322) et dudit segment de plus petit diamètre (214) est formé avec une rainure de retenue (223), et l'autre dudit segment inférieur (322) et dudit segment de plus petit diamètre (214) est formé avec une protubérance de retenue (327) qui s'engage avec faculté de retenue avec ladite rainure de retenue (223) lorsque ledit segment inférieur (322) est amené vers la position d'utilisation de façon à générer la première force de frottement.

3. Seringue jetable selon la revendication 1, **caractérisée en outre en ce que** ledit segment de retenue a une portion concave (326) qui s'étend vers l'intérieur de ladite surface de paroi tubulaire interne (324) dans des directions radiales par rapport à l'axe pour former une seconde portion d'épaulement environnante (329) qui est adjacente à ladite portion de bord environnante inférieure (33), ladite tête (50b) ayant une portion de butée environnante (541, 741) qui est configurée de façon à être insérable dans ladite portion concave (326) et pour venir en butée contre ladite seconde portion d'épaulement environnante (329), générant ainsi la quatrième force de frottement pour maintenir ledit siège d'aiguille (30) à la position rétractée.

4. Seringue jetable selon la revendication 3, **caractérisée en outre en ce que** ladite tête (50b) a une portion de fuseau (55) qui s'étend depuis ladite portion de butée environnante (541) le long de l'axe (X) et qui se termine en deux moitiés partagées (552, 553) qui sont espacées l'une de l'autre dans une direction transversale à la direction longitudinale, lesdites moitiés partagées (552, 553) ayant respectivement deux bords d'ancrage terminaux supérieurs (554, 555) qui sont éloignés de ladite portion de butée environnante (541) qui sont configurés de telle sorte que lesdites moitiés partagées (552, 553) sont pressées par ladite surface de paroi tubulaire interne (324) pour se déplacer l'une vers l'autre contre une action de sollicitation lorsque lesdites moitiés partagées (552, 553) sont déplacées vers ladite portion de bord environnante supérieure (321), et de telle sorte que lesdits bords d'ancrage terminaux supérieurs (554, 555) s'engagent avec ladite portion de bord environnante supérieure (321) une fois que lesdites moitiés partagées (552, 553) sont déplacées au-delà de ladite portion de bord environnante supérieure (321).

5. Seringue jetable selon la revendication 4, **caractérisée en outre en ce que** ladite surface de paroi tubulaire interne (324) a un diamètre qui diminue progressivement de ladite portion de bord environnante inférieure (33) à ladite portion de bord environnante supérieure (321), ladite portion de fuseau (55) ayant un diamètre externe qui diminue progressivement de ladite portion de butée environnante (541) auxdites moitiés partagées (552, 553).

6. Seringue jetable selon la revendication 3, **caractérisée en outre en ce que** ladite portion d'engagement environnante (60) est constituée d'un matériau déformable, et a des faces d'extrémité supérieure et inférieure (64, 65) opposées l'une à l'autre dans la direction longitudinale, à proximité de et distales à ladite portion de bord environnante inférieure (33), respectivement, et une surface environnante externe (63) disposée entre lesdites faces d'extrémité supérieure et inférieure (64, 65) et concave vers l'axe (X).

7. Seringue jetable selon la revendication 1, **caractérisée en outre en ce que** ladite portion de bord environnante inférieure (33) a une surface de bord supérieure (331) qui vient en butée contre ladite première portion d'épaulement environnante (215) lorsque ledit segment inférieur (322) est à la position d'utilisation, et une surface de bord inférieure (332) pour butée et enfoncement par ladite portion d'engagement environnante (60).

8. Seringue jetable selon la revendication 1, **caractérisée en outre par** un organe de cannelure (216, 328) disposé entre ledit segment de petit diamètre (214) et ledit segment inférieur (322) pour empêcher une rotation relative entre eux.

9. Seringue jetable selon la revendication 1, **caractérisée en outre par** une bague de protubérance (217) qui est disposée sur et qui fait saillie vers l'intérieur et radialement dudit segment de plus grand diamètre (213) adjacent à ladite extrémité ouverte inférieure (211), et une bride environnante (51) qui est disposée pour entourer ledit segment interne (502) adjacent à ladite tête (50b) et qui est engageable avec ladite bague de protubérance (217).

10. Seringue jetable selon la revendication 9, **caractérisée en outre en ce que** ledit segment interne (502) a une zone d'affaiblissement (531, 731) qui est disposée à proximité de ladite bride environnante (51) de telle sorte que lorsque ladite bride environnante (51) est amenée à s'engager avec ladite bague de protubérance (217), ladite zone d'affaiblissement (531, 731) s'étend vers l'extérieur de ladite extrémité ouverte inférieure (211) de façon à faciliter la cassure de ladite portion de tige (50a, 73) hors dudit plongeur (50, 70) au niveau de ladite zone d'affaiblissement (531, 731).

11. Seringue jetable selon la revendication 3, **caractérisée en outre en ce que** ladite portion de butée environnante (541, 741) est configurée pour être sensiblement creuse pour une plus grande flexibilité.

12. Seringue jetable selon la revendication 1, **caractérisée en outre en ce que** ledit tube (20) a une surface de paroi environnante externe (222) disposée à l'opposé de ladite surface de paroi de tube environnante interne (221) dans des directions radiales et ayant une portion manchonnée (2221) qui est disposée à proximité de ladite extrémité ouverte supérieure (212), ladite seringue jetable comprenant en outre un protecteur d'embout (43) qui a une extrémité de manchon interne (433) configurée pour être manchonnée sur ladite portion manchonnée (2221) de façon à protéger l'aiguille (41) de l'unité d'aiguille (40).

13. Seringue jetable selon la revendication 12, **caractérisée en outre en ce que** chacune de ladite portion manchonnée (2221) et ladite extrémité de manchon interne (433) a une pluralité de nervures de frottement (224, 433) formée sur celle-ci.

14. Seringue jetable selon la revendication 13, **caractérisée en outre en ce que** ladite extrémité ouverte supérieure (212) a une paroi de bord environnante (219) qui s'étend dudit segment de plus petit diamètre (214) de ladite surface de paroi de tube environnante (221) vers l'axe (X) et qui confine un trou traversant (210) qui entoure l'axe (X) et est adapté pour un passage de l'aiguille (41) de l'unité d'aiguille (40).

15. Seringue jetable selon la revendication 1, **caractérisée en outre en ce que** ledit segment supérieur (323) et ladite portion de bord environnante supérieure (321) s'étendent vers l'extérieur de ladite extrémité ouverte supérieure (212) lorsque ledit segment inférieur (322) est dans la position d'utilisation, ladite seringue jetable comprenant en outre un pavillon d'aiguille (42) qui est adapté pour attacher l'aiguille (41) de l'unité d'aiguille (40), qui est disposé pour se manchonner sur ledit segment supérieur (323) et qui a une portion manchonnée (423), et un protecteur d'embout (43) qui a une extrémité de manchon interne (433) configurée pour être manchonnée sur ladite portion manchonnée (423) de façon à protéger l'aiguille (41).
